Europäisches Patentamt

European Patent Office

Office européen des brevets

(19)

(11) Veröffentlichungsnummer: **0 035 703**
**B1**

(12) **EUROPÄISCHE PATENTSCHRIFT**

(45) Veröffentlichungstag der Patentschrift:
28.09.83

(21) Anmeldenummer: **81101397.8**

(22) Anmeldetag: **26.02.81**

(51) Int. Cl.³: **C 07 C 33/14**, C 07 C 29/00,
B 01 F 17/42, B 01 J 27/16

(54) Verfahren zur Herstellung von Terpineol.

(30) Priorität: **10.03.80 AT 1309/80**

(43) Veröffentlichungstag der Anmeldung:
**16.09.81 Patentblatt 81/37**

(45) Bekanntmachung des Hinweises auf die Patenterteilung:
**28.09.83 Patentblatt 83/39**

(84) Benannte Vertragsstaaten:
**CH DE FR GB LI**

(56) Entgegenhaltungen:
**CH-A-92 123**
**DE-A-1 468 045**
**DE-C-692 416**
**US-A-2 178 349**

**Die Akte enthält technische Angaben, die nach dem
Eingang der Anmeldung eingereicht wurden und die
nicht in dieser Patentschrift enthalten sind.**

(73) Patentinhaber: **Krems-Chemie Gesellschaft m.b.H.,
Hafenstrasse 77, A-3500 Krems (AT)**

(72) Erfinder: **Charwath, Michael, Dr.,
Heinemannstrasse 6a/6, A-3500 Krems (AT)**

(74) Vertreter: **Mann, Volker, Dr. et al, c/o Bayer
Aktiengesellschaft Zentralbereich Patente Marken und
Lizenzen, D-5090 Leverkusen-Bayerwerk (DE)**

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren zur Herstellung von Terpineol

Die vorliegende Erfindung betrifft ein Verfahren zur Herstellung von Terpineol durch direkte Hydratisierung von Pinen und/oder Pinen enthaltenden Rohstoffen.

Terpineol wird aufgrund seines anhaltend angenehmen Geruches als Riechstoff oder in Form von Pineoil für die verschiedensten Lösungs-, Reinigungs- und Desinfektionsmittel verwendet. Seine Herstellung wurde bisher hauptsächlich in einem zweistufigen Verfahren durchgeführt.

Bei diesem bekannten Verfahren wird in der ersten Stufe $\alpha$- oder $\beta$-Pinen oder ein mindestens eine dieser Verbindungen enthaltender Rohstoff, wie Balsam-, Sulfat- oder Holzterpentinöl, mit anorganischen Säuren hydratisiert und das gebildete Terpinhydrat von der Säure und dem ebenfalls entstandenen Nebenöl abgetrennt. Unter den angewendeten Säuren sind vor allem Schwefelsäure, Salpetersäure und Phosphorsäure zu nennen, von denen bisher jedoch nur Schwefelsäure großtechnische Anwendung gefunden hat. In der zweiten Stufe wird das Terpinhydrat mit verdünnten Säuren partiell zu Terpineol dehydratisiert. Zum Einsatz gelangen hier Schwefelsäure, Phosphorsäure und zahlreiche organische Säuren, wie z. B. die in US-PS 2 336 575 genannte Oxalsäure, Phthalsäure oder Weinsäure.

Die Abtrennung des Terpinhydrates und insbesondere die bei der Dehydratisierung notwendige Entfernung des gebildeten Terpineols durch Wasserdampfdestillation stellen zwei äußerst arbeits- und energieintensive Verfahrensschritte dar. Auch die bei der Dehydratisierung in beträchtlichem Umfang als Nebenreaktion eintretende und die Alkoholausbeute schmälernde Kohlenwasserstoffbildung sind bei diesem Verfahren nachteilig.

Es wurden deshalb Versuche unternommen, die Hydratisierungsreaktion auf der Zwischenstufe des Terpineols zu stoppen und so in einem Einstufenverfahren direkt aus Pinen Terpineol zu erhalten.

In der US-PS 2 178 349 wird eine Methode zur Hydratisierung von Terpentinöl in acetonischer oder dioxanischer Lösung mit 32%iger Schwefelsäure angegeben, wobei die Bildung von Terpinhydrat beinahe vollständig unterbleibt. In der SU-PS 241 423 wird ebenfalls Aceton als Lösungsmittel empfohlen. Die Verwendung von Aceton in Kombination mit Amidoschwefelsäure wird in US-PS 2 432 556 vorgeschlagen.

Die PL-PS 50 391 lehrt, daß beim Einsatz von schwefliger Säure als Katalysator Ausbeuten bis zu 70% der Theorie an Alkoholen, allerdings nur bei stark verlängerter Reaktionszeit, möglich sind.

Ein völlig anderer Weg wird in der US-PS 3 281 479 durch Verwendung von Essigsäure in Gegenwart von Bortrifluorid in Kombination mit einem Cokatalysator vorgeschlagen. Die Verwendung von Chloressigsäure wird in JP-PS 39 118 beschrieben. In der JP-PS 110 267 wird die Wirkung von Kationenaustauschern erläutert.

Alle diese Verfahren, außer denjenigen mit den relativ kostspieligen Kationenaustauschern, haben entweder den Nachteil des Arbeitens in sehr verdünnter Lösung, wobei große Mengen eines Lösungsmittels abdestilliert werden müssen und geringe Restgehalte dieses Lösungsmittels die Qualität des Endproduktes mindern können, oder den Nachteil der Anwendung stark korrodierender, meist teurer Säuren, die überdies im allgemeinen nur durch sehr aufwendige Aufbereitung einem erneuten Einsatz in die Reaktion zugeführt werden können.

Aus der DE-OS 1 468 045 ist ein Verfahren zur Herstellung von Terpenalkoholen und synthetischem Pine Oil bekannt, bei dem $\alpha$-Pinen enthaltendes Terpenmaterial mit bestimmten Mengen wäßriger Schwefelsäure von 35 bis 45 Gew.-% bei 35 bis 45° C und in Gegenwart eines Emulgators gerührt wird. Nachteilig bei diesem Verfahren ist, daß man beim Alkoholspitzengehalt des Reaktionsgemisches ein Rohöl erhält, das hinsichtlich der Ausbeute an Alkoholen in % der Theorie, bezogen auf umgesetztes $\alpha$-Pinen, hinsichtlich des Gehaltes an Alkoholen und hinsichtlich des Gehaltes an $\alpha$-Terpineol unbefriedigend ist.

Es wurde nun ein Verfahren zur Herstellung von Terpineol durch direkte Hydratisierung von $\alpha$- und/oder $\beta$-Pinen und/oder eines mindestens eine dieser Verbindungen enthaltenden Rohstoffes zu Terpineol mit wäßriger Mineralsäure in Gegenwart eines Emulgators gefunden, das dadurch gekennzeichnet ist, daß man die Hydratisierung des Ausgangsproduktes mit 20- bis 60gew.-%iger wäßriger Phosphorsäure vornimmt, wobei man mindestens 0,5 Vol.-Teile Phosphorsäure auf 1 Vol.-Teil Ausgangsprodukt einsetzt, in Gegenwart von 0,05 bis 0,5 Gew.-% (bezogen auf das Gewicht der Phosphorsäure) eines nicht-ionogenen oder kationaktiven Emulgators arbeitet, die organische und die wäßrige Phase gut durchmischt und Temperaturen im Bereich 40 bis 80° C einhält.

Der Reaktionsverlauf des erfindungsgemäßen Verfahrens wird hauptsächlich durch folgende Parameter bestimmt:

— Art und Konzentration der Säure
— Volumen- oder Gewichtsverhältnis von Säure zu Ausgangsprodukt
— Art und Menge des Emulgators
— Grad der Homogenisierung
— Reaktionstemperatur

Durch die erfindungsgemäße Kombination dieser Reaktionsparameter kann die Terpinhydratbildung weitgehend oder vollkommen unterdrückt werden.

Als Ausgangsprodukte für das erfindungsgemäße Verfahren können $\alpha$-Pinen und/oder $\beta$-Pinen, beispielsweise jeweils in reiner Form oder als technische Produkte mit Gehalten an $\alpha$- und/oder $\beta$-Pinen von beispielsweise über 80%, sowie mindestens eine dieser Verbindungen enthaltende Rohstoffe, beispielsweise Balsam-, Sulfat- oder Holzterpentinöl eingesetzt werden, die beispielsweise über 60% $\alpha$-Pinen enthalten können.

Ein wesentliches Merkmal des erfindungsgemäßen Verfahrens ist die Verwendung von 20- bis 60gew.-%iger wäßriger Phosphorsäure für die direkte Hydratisierung von Pinen und/oder Pinene enthaltenden Ausgangsprodukten zu Terpineol. Die Phosphorsäure wird dabei in Mengen von mindestens 0,5 Vol.-Teilen pro Vol.-Teil Ausgangsprodukt eingesetzt. Nach oben hin ist die in das erfindungsgemäße Verfahren einsetzbare Phosphorsäuremenge nicht kritisch. Aus praktischen und wirtschaftlichen Erwägungen arbeitet man vorzugsweise jedoch nicht mit mehr als 3 Vol.-Teilen Phosphorsäure pro Vol.-Teil Ausgangsprodukt. Besonders bevorzugt werden 0,8 bis 1,5 Vol.-Teile, ganz besonders bevorzugt etwa 1 Vol.-Teil Phosphorsäure pro Vol.-Teil Ausgangsprodukt eingesetzt.

Beispiele für einsetzbare nicht-ionogene Emulgatoren sind Nonylphenol-ethoxylate und Fettalkohol-ethoxylate.

Vorzugsweise werden Emulgatoren in Mengen von 0,05 bis 0,3 Gew.-%, bezogen auf die Phosphorsäure, eingesetzt.

Von besonderem Vorteil ist der Einsatz von nicht-ionogenen Emulgatoren auf Ethylenoxidbasis, die bei der angewendeten Reaktionstemperatur von der Säure langsam zersetzt werden. Nach Beendigung der Reaktion und Abstellen des Rührwerkes kommt es dann bei entsprechendem Fortschritt des Abbaus der Ethylenoxidkette zu einer raschen Trennung zwischen wäßriger und organischer Phase. Ein derartiges Verhalten konnte bei Reaktionstemperaturen zwischen 40 und 60°C insbesondere bei Emulgatoren auf Ethylenoxidbasis mit einem Trübungspunkt von 40 bis 65°C (Messung 2%ig in Wasser) und bei Reaktionstemperaturen zwischen 60 und 80°C insbesondere bei Emulgatoren auf Ethylenoxidbasis mit einem Trübungspunkt von 65 bis 80°C (gemessen 2%ig in 10%iger Kochsalzlösung) festgestellt werden.

Die Verwendung stabilerer oberflächenaktiver Substanzen führt ebenso zu guten Ausbeuten an Terpineol, doch kann dann im allgemeinen eine vollständige Trennung der Emulsion nach Beendigung der Reaktion nur nach teilweiser Neutralisation der Phosphorsäure erreicht werden.

Die erfindungsgemäß notwendige gute Durchmischung der organischen und wäßrigen Phase während der Reaktion kann durch die Verwendung von Reaktionsgefäßen erreicht werden, die dies gestatten, beispielsweise durch die Verwendung von mit Rührwerken versehenen Reaktoren.

Das erfindungsgemäße Verfahren wird bei Temperaturen im Bereich 40 bis 80°C durchgeführt. Es ist vorteilhaft, innerhalb dieses Bereiches bei relativ hohen Temperaturen zu arbeiten, beispielsweise im Bereich 60 bis 80°C, wenn die Konzentration der eingesetzten Phosphorsäure relativ niedrig, beispielsweise 20- bis 40gew.-%ig ist. Relativ niedrige Reaktionstemperaturen, beispielsweise 40 bis 60°C, werden vorzugsweise dann angewendet, wenn die Konzentration der Phosphorsäure relativ hoch, beispielsweise 40- bis 60gew.-%ig, ist.

Die Reaktionszeit, d. h. die Zeit, die notwendig ist, bis das eingesetzte Pinen vollständig oder bis zum gewünschten Ausmaß reagiert hat, ist um so kürzer, je höher konzentriert die Phosphorsäure und je höher die Reaktionstemperatur eingestellt worden ist. Die Reaktionszeit beträgt, je nach den gewählten Bedingungen, im allgemeinen zwischen 2 und 30 Stunden.

Es kann vorteilhaft sein, die Reaktion nicht bis zur vollständigen Umsetzung des eingesetzten Pinens durchzuführen, sondern bei einem bestimmten Restgehalt an Pinen im Rohöl, abzubrechen. Beispielsweise kann dieser Gehalt an Pinen im Rohöl im Bereich 1 bis 35 Gew.-% liegen. In diesen Fällen kann das bei der Aufarbeitung des Rohöls anfallende, unumgesetzte Pinen erneut dem erfindungsgemäßen Verfahren zugeführt werden. Auf diese Weise sind Alkoholausbeuten bis zu etwa 70% der Theorie, bezogen auf umgesetztes Pinen, möglich.

Die Aufarbeitung des nach der Reaktion vorliegenden Gemisches kann auf einfache Weise erfolgen. Beispielsweise kann man dabei wie folgt vorgehen: Man trennt zunächst die wäßrige, Phosphorsäure enthaltende Phase von der organischen Phase. Hier kann es, beispielsweise bei Verwendung stabiler Emulgatoren, notwendig werden, einen Teil der vorliegenden Säure zu neutralisieren. Die abgetrennte wäßrige Phosphorsäure kann, vorzugsweise nach Einstellung der ursprünglichen Konzentration und Ersatz der geringen an der organischen Phase anhaftenden Mengen, wieder im erfindungsgemäßen Verfahren verwendet werden. Wenn, beispielsweise bei Verwendung von Phosphorsäure höherer Konzentration und/oder nicht vollständiger Umsetzung des Pinens, beim längeren Stehen der gebrauchten Phosphorsäure geringe Mengen an Terpinhydrat auskristallisieren, hat dies keinen negativen Einfluß auf die Wiederverwendbarkeit der Säure, da dieses Terpinhydrat beim Wiedereinsatz zu Terpineol dehydratisiert wird.

Die abgetrennte organische Phase, das sogenannte Rohöl, enthält je nach den angewendeten Reaktionsbedingungen bis zu etwa 50% Terpenalkohole. Das Rohöl wird im allgemeinen nach Neutralisation, beispielsweise mit einer verdünnten wäßrigen Alkali- oder Sodalösung, bei vermindertem Druck rektifiziert. Gegebenenfalls kann dabei vor dem Überdestillieren der Alkohole

3

eine Pinen- und/oder eine Terpenkohlenwasserstofffraktion abgetrennt werden.

Das erfindungsgemäße Verfahren kann so durchgeführt werden, daß nach der Reaktion kein Terpinhydrat vorliegt, was allerdings im allgemeinen relativ lange Reaktionszeiten erfordert. Es kann von Vorteil sein, die Bildung geringer Mengen von Terpinhydrat zuzulassen, wodurch dann die Reaktionszeit im allgemeinen stark verkürzt werden kann. Auch in solchen Fällen kristallisiert im allgemeinen während der Reaktion und nach der Beendigung der guten Durchmischung kein Terpinhydrat aus, so daß die Trennung der wäßrigen von der organischen Phase problemlos möglich ist. Eine Auskristallisation von wenig Terpinhydrat erfolgt in solchen Fällen im allgemeinen erst bei der Neutralisation des Rohöls. Die Kristalle können dort mit einfachen Mitteln, beispielsweise durch Filtration, abgetrennt werden.

Das erfindungsgemäße Verfahren hat eine Reihe von Vorteilen. So sind zunächst die nicht-korrosierenden Eigenschaften der als Hydratisierungsreagenz verwendeten Phosphorsäure anzuführen, die insbesondere bei höheren Temperaturen von Interesse sind. Weiterhin gelingt es, Terpineol in besseren Selektivitäten zu erhalten, als dies in dem bekannten Zweistufenverfahren über Terpinhydrat als Zwischenprodukt oder in den bekannten, in homogener Lösung durchgeführten Einstufenverfahren möglich ist. Ein weiterer Vorteil ist die leichte Durchführbarkeit des erfindungsgemäßen Verfahrens unter Verwendung einfacher Apparate und ohne zusätzliche Lösungsmittel. Beispielsweise können Apparate verwendet werden, die bisher für die Herstellung von Terpinhydrat eingesetzt wurden. Schließlich kann sowohl die Phosphorsäure als auch gegebenenfalls nichtumgesetztes Pinen und/oder geringe Mengen gegebenenfalls gebildetes Terpinhydrat wieder eingesetzt werden.

Die folgenden Beispiele erläutern die Erfindung. Die Angaben in Prozent der Theorie beziehen sich auf umgesetztes Pinen, da nichtumgesetztes Pinen in die Reaktion zurückgeführt werden kann.

## Beispiele

Die in den Beispielen verwendeten Emulgatoren waren folgende:

Emulgator A: Ein $C_{18}$-Fettalkohol mit einer Jodzahl von 50 mit 20 Mol Ethylenoxid. Der Trübungspunkt betrug, gemessen als 2%ige Lösung in 10%iger Kochsalzlösung, 74 bis 78° C.

Emulgator B: Ein Nonylphenolether mit 9 Mol Ethylenoxid. Der Trübungspunkt betrug, gemessen als 2%ige Lösung in Wasser, 48 bis 50° C.

Die Zusammensetzung der erhaltenen Rohöle und eine Zusammenfassung der erhaltenen Ausbeuten ist aus Tabelle 1 ersichtlich.

## Beispiel 1

1000 ml 25%ige wäßrige Phosphorsäure wurden mit 1,15 g Emulgator A versetzt und auf 70° C erwärmt. Bei Erreichen dieser Temperatur wurden 1000 ml $\alpha$-Pinen (95%ig) zugesetzt. Die Rührleistung wurde so eingestellt, daß eine vollständige Einmischung der organischen Phase gewährleistet war.

Die Reaktionstemperatur wurde während 24 Stunden auf 70 ± 1° C gehalten. Anschließend ließ man die Emulsion in ein Trenngefäß laufen, wo die Abtrennung der Säure vom Rohöl in wenigen Minuten erfolgte. Das Rohöl wurde mit verdünnter Sodalösung neutralisiert und anschließend rektifiziert. Es entstand praktisch kein Terpinhydrat.

Ausbeute an Rohöl: 850 g.

## Beispiel 2

1000 ml 30%ige wäßrige Phosphorsäure, 1,19 g Emulgator A und 1000 ml $\alpha$-Pinen (95%ig) wurden in der in Beispiel 1 genannten Reihenfolge und Vorgangsweise gemischt. Dann wurde während 16 Stunden eine Reaktionstemperatur von 70 ± 1° C aufrechterhalten. Die Aufarbeitung des Reaktionsgemisches erfolgte gemäß der im Beispiel 1 genannten Arbeitsweise Es entstand kein Terpinhydrat.

Ausbeute an Rohöl: 835 g.

## Beispiel 3

1000 ml 40%ige wäßrige Phosphorsäure, 1,26 g Emulgator A und 1000 ml $\alpha$-Pinen (95%ig) wurden bei 60° C gemischt und bei dieser Temperatur 24 Stunden unter inniger Durchmischung gerührt. Nach Abtrennung der Säure wurde das Rohöl mit verdünnter Sodalösung neutralisiert. Es bildete sich kein Terpinhydrat. Das neutralisierte Rohöl wurde rektifiziert.

Ausbeute an Rohöl: 820 g.

## Beispiel 4

1000 ml 50%ige wäßrige Phosphorsäure und 2,66 g Emulgator B wurden auf 50°C erwärmt. Bei Erreichen dieser Temperatur wurden 1000 ml $\alpha$-Pinen (95%ig) zugesetzt. Unter inniger Durchmischung beider Phasen wurde die Reaktionstemperatur während 6 Stunden auf $50 \pm 1$°C gehalten.

Anschließend wurde die Säure vom Rohöl getrennt und dieses mit verdünnter Sodalösung neutralisiert. Während der Neutralisation des Rohöls kristallisierten geringe Mengen Terpinhydrat aus. Aus der abgetrennten Säure konnte kein Terpinhydrat gewonnen werden. Das neutralisierte Rohöl wurde rektifiziert.

Ausbeute an Rohöl: 815 g.
Ausbeute an Terpinhydrat: 26,5 g.

## Beispiel 5

Entsprechend der Vorgangsweise von Beispiel 4 wurde 1000 ml 50%ige wäßrige Phosphorsäure, 2,66 g Emulgator B und 1000 ml $\alpha$-Pinen (95%ig) zur Reaktion gebracht. Die Reaktion wurde jedoch bereits nach 4 Stunden abgebrochen. Die Aufarbeitung erfolgte in der in Beispiel 4 genannten Arbeitsweise. Bei der Neutralisation des Rohöls trat geringfügige Kristallisation von Terpinhydrat auf. Nach 48stündigem Stehen konnten auch geringe Mengen an Terpinhydrat aus der Säure gewonnen werden. Das neutralisierte Rohöl wurde rektifiziert.

Ausbeute an Rohöl: 817 g.
Ausbeute an Terpinhydrat: 53,2 g.

## Beispiel 6

1000 ml 60%ige wäßrige Phosphorsäure, 1,43 g Emulgator B und 1000 ml $\alpha$-Pinen (95%ig) wurden bei 40°C gemischt und 4 Stunden unter inniger Durchmischung bei dieser Temperatur gerührt.

Anschließend wurde die Reaktionsmischung von etwas gebildetem Terpinhydrat abgesaugt, die Säure vom Rohöl getrennt und dieses mit verdünnter Sodalösung neutralisiert. Dabei kam es zu einer zweiten Kristallisation von Terpinhydrat, welches durch Filtration abgetrennt wurde. Das neutralisierte Rohöl wurde rektifiziert.

Ausbeute an Rohöl: 752 g.
Ausbeute an Terpinhydrat: 96,8 g.

## Beispiel 7

Es wurde wie in Beispiel 4 gearbeitet, jedoch wurden anstelle von 1000 ml $\alpha$-Pinen 1000 ml Balsam-Terpentinöl mit einem Gehalt an $\alpha$-Pinen von 64% eingesetzt. Die Ausbeute an Rohöl betrug 863 g, diejenige an Terpinhydrat 8,2 g.

## Beispiel 8

Es wurde verfahren wie in Beispiel 4 beschrieben, jedoch wurden anstelle von 1000 ml $\alpha$-Pinen 1000 ml Sulfat-Terpentinöl mit einem Gehalt an $\alpha$-Pinen von 64% eingesetzt. Die Ausbeute an Rohöl betrug 884 g, diejenige an Terpinhydrat 7,8 g.

Tabelle 1

Ausbeuteschema der Beispiele 1 bis 8

| | Beispiel Nr. | | | | | | | |
|---|---|---|---|---|---|---|---|---|
| | 1 | 2 | 3 | 4 | 5 | 6 | 7 | 8 |
| Zusammensetzung des Rohöles in Gewichtsprozent | | | | | | | | |
| α-Pinen | 25,8 | 25,1 | 0,5 | 10,5 | 32,7 | 32,6 | 5,6 | 12,5 |
| monocyclische Monoterpen-kohlenwasserstoffe | 28,2 | 32,5 | 60,4 | 41,5 | 30,8 | 33,3 | 44,8 | 49,4 |
| Terpenalkohole | 46,0 | 42,4 | 38,1 | 48,0 | 36,5 | 34,1 | 43,8 | 38,1 |
| Ausbeute an Alkoholen in Prozent der Theorie[1]) | 57,8 | 51,5 | 33,9 | 47,2 | 47,9 | 45,1 | 66,8 | 67,8 |
| Ausbeute an Terpinhydrat in Prozent der Theorie[1]) | — | — | — | 2,5 | 6,9 | 13,8 | 1,1 | 1,2 |

[1]) bezogen auf umgesetztes α-Pinen.

**Patentansprüche**

1. Verfahren zur Herstellung von Terpineol durch direkte Hydratisierung von α- und/oder β-Pinen und/oder eines mindestens eine dieser Verbindungen enthaltenden Rohstoffes zu Terpineol mit wäßriger Mineralsäure in Gegenwart eines Emulgators, dadurch gekennzeichnet, daß man die Hydratisierung des Ausgangsproduktes mit 20- bis 60gew.-%iger wäßriger Phosphorsäure vornimmt, wobei man mindestens 0,5 Vol.-Teile Phosphorsäure auf 1 Vol.-Teil Ausgangsprodukt einsetzt, in Gegenwart von 0,05 bis 0,5 Gew.-% (bezogen auf das Gewicht der Phosphorsäure) eines nicht-ionogenen oder kationenaktiven Emulgators arbeitet, die organische und die wäßrige Phase gut durchmischt und Temperaturen im Bereich 40 bis 80°C einhält.

2. Verfahren nach Anspruch 1, dadurch gekennzeichnet, daß man nicht mehr als 3 Vol.-Teile Phosphorsäure auf 1 Vol.-Teil Ausgangsprodukt einsetzt.

3. Verfahren nach Anspruch 1 und 2, dadurch gekennzeichnet, daß man als nicht-ionogene Emulgatoren Nonylphenol-Ethoxylate oder Fettalkohol-Ethoxylate einsetzt.

4. Verfahren nach Anspruch 1 bis 3, dadurch gekennzeichnet, daß man nicht-ionogene Emulgatoren auf Ethylenoxidbasis einsetzt, die bei der angewendeten Reaktionstemperatur von der Säure langsam zersetzt werden.

5. Verfahren nach Anspruch 1 bis 4, dadurch gekennzeichnet, daß man bei Temperaturen im Bereich von 60 bis 80°C und mit 20- bis 40gew.-%iger wäßriger Phosphorsäure arbeitet.

6. Verfahren nach Anspruch 1 bis 4, dadurch gekennzeichnet, daß man bei Temperaturen im Bereich von 40 bis 60°C und mit 40- bis 60gew.-%iger wäßriger Phosphorsäure arbeitet.

7. Verfahren nach Anspruch 1 bis 6, dadurch gekennzeichnet, daß man die Hydratisierung durchführt, bis das eingesetzte Pinen vollständig reagiert hat.

8. Verfahren nach Anspruch 1 bis 6, dadurch gekennzeichnet, daß man die Hydratisierung durchführt, bis der Gehalt an Pinen im Rohöl 1 bis 35 Gew.-% beträgt.

**Claims**

1. Process for the preparation of terpineol by direct hydration of α- and/or β-pinene and/or of a raw material containing at least one of these compounds to give terpineol using aqueous mineral acid in the presence of an emusifier, characterised in that the hydration of the starting product is carried out using 20 to 60% strength, by weight, aqueous phosphoric acid, at least 0.5 parts by volume of phosphoric acid being used per 1 part by volume of starting product, the process is carried out in the presence of 0.05 to 0.5%, by weight, (based on the weight of the phosphoric acid) of a non-ionogenic or cationic emulsifier, the organic and the aqueous phases are mixed well and temperatures are maintained in the range from 40 to 80°C.

2. Process according to Claim 1, characterised in that not more than 3 parts by volume of phosphoric acid are used per 1 part by volume of starting product.

3. Process according to Claim 1 and 2, characterised in that nonylphenyl ethoxylates or aliphatic alcohol ethoxylates are used as non-ionogenic emulsifiers.

4. Process according to Claim 1 to 3, characterised in that non-ionogenic emulsifiers based on ethylene oxide are used which are slowly decomposed by the acid at the reaction temperature used.

5. Process according to Claim 1 to 4, characterised in that it is carried out at temperatures in the range from 60 to 80°C and using 20 to 40% strength, by weight, aqueous phosphoric acid.

6. Process according to Claim 1 to 4, characterised in that it is carried out at temperatures in the range from 40 to 60°C and using 40 to 60% strength, by weight, aqueous phosphoric acid.

7. Process according to Claim 1 to 6, characterised in that the hydration is carried out until the pinene used has reacted completely.

8. Process according to Claim 1 to 6, characterised in that the hydration is carried out until the content of pinene in the crude oil is 1 to 35% by weight.


**Revendications**

1. Procédé de production de terpinéol par hydratation directe en terpinéol de l'$\alpha$- et/ou du $\beta$-pinène et/ou d'une matière première contenant au moins l'un de ces composés, avec un acide minéral aqueux en présence d'un émulsionnant, caractérisé en ce qu'on effectue l'hydratation de la matière de départ avec de l'acide phosphorique aqueux à 20–60% en poids, en utilisant alors au moins 0,5 partie en volume d'acide phosphorique par partie en volume de matière de départ, on opère en présence de 0,05 à 0,5% en poids (par rapport au poids d'acide phosphorique) d'un émulsionnant non ionogène ou doué d'activité cationique, on mélange correctement la phase organique et la phase aqueuse et on maintient les températures dans la plage de 40 à 80°C.

2. Procédé suivant la revendication 1, caractérisé en ce qu'on n'utilise pas plus de 3 parties en volume d'acide phosphorique par partie en volume de matière de départ.

3. Procédé suivant les revendications 1 et 2, caractérisé en ce qu'on utilise comme émulsionnants non ionogènes des éthoxylates de nonylphénol ou des éthoxylates d'alcools gras.

4. Procédé suivant les revendications 1 à 3, caractérisé en ce qu'on utilise des émulsionnants non ionogènes à base d'oxyde d'éthylène, qui sont décomposés lentement par l'acide à la température de réaction utilisée.

5. Procédé suivant les revendications 1 à 4, caractérisé en ce qu'on opère à des températures comprises dans la plage de 60 à 80°C et avec de l'acide phosphorique aqueux à 20–40% en poids.

6. Procédé suivant les revendications 1 à 4, caractérisé en ce qu'on opère à températures comprises dans la plage de 40 à 60°C et avec de l'acide phosphorique aqueux à 40–60% en poids.

7. Procédé suivant les revendications 1 à 6, caractérisé en ce qu'on conduit l'hydratation jusqu'à ce que le pinène utilisé ait totalement réagi.

8. Procédé suivant les revendications 1 à 6, caractérisé en ce qu'on conduit l'hydratation jusqu'à ce que la teneur en pinène dans l'huile brute s'élève à 1–35% en poids.